# EUROPÄISCHE PATENTSCHRIFT

(11) **EP 1 040 190 B1**
(45) Veröffentlichungstag und Bekanntmachung des Hinweises auf die Patenterteilung: **06.04.2005**
(21) Anmeldenummer: 98966667.2
(22) Anmeldetag: 23.12.1998
(51) Int. Cl.: C12N 15/12, C12N 15/11, C07K 14/81, C07K 16/38, A61K 31/70, A61K 38/57, A61K 48/00, G01N 33/68, C07H 21/04

(54) **SERIN-PROTEINASE-INHIBITOREN**
SERINE PROTEINASE INHIBITORS
INHIBITEURS DE SERINE-PROTEINASE

(30) Priorität: 23.12.1997 DE 19757572; 08.01.1998 DE 19800363
(43) Veröffentlichungstag der Anmeldung: 04.10.2000
(73) Patentinhaber: Pharis Biotec GmbH, 30625 Hannover (DE)
(72) Erfinder: FORSSMANN, Wolf-Georg, D-30625 Hannover (DE); MÄGERT, Hans-Jürgen, D-30163 Hannover (DE); STÄNDKER, Ludger, D-30625 Hannover (DE); KREUTZMANN, Peter, D-39116 Magdeburg (DE)
(74) Vertreter: Meyers, Hans-Wilhelm, Dr.Dipl.-Chem.
(86) Internationale Anmeldenummer: PCT/EP1998/008424
(87) Internationale Veröffentlichungsnummer: WO 1999/033974

(56) Entgegenhaltungen:
- EP-A- 0 300 459
- WO-A-95/02055
- WO-A-97/15670

## Beschreibung

Die Erfindung betrifft Serin-Proteinase-Inhibitoren, cDNA kodierend für Serin-Proteinase-Inhibitoren, Arzneimittel enthaltend die Inhibitoren oder deren codierende Nucleinsäure, Verwendungen der erfindungsgemäßen Verbindungen zur Herstellung von Arzneimitteln zur Behandlung verschiedener Indikationen, Antikörper oder Antikörperfragmente gegen Epitope der erfindungsgemäßen Verbindungen sowie Poly- oder Oligonucleotide, die mit Genen der erfindungsgemäßen Verbindungen hybridisieren.

Proteolytische Prozesse spielen in allen Organismen eine bedeutende physiologische Rolle, wobei zwischen unspezifischen und spezifischen proteolytischen Reaktionen zu unterscheiden ist. Zu den ersten gehören beispielsweise der Nahrungsaufschluß im Verdauungstrakt durch Endopeptidasen sowie der intrazelluläre Abbau verbrauchter endogener Substanzen und phagozytierten Materials durch lysosomale Proteinasen. Spezifische Proteolysen dienen meistens der Überführung eines Proenzyms in die aktive Form wie bei der Überführung von Trypsinogen in Trypsin und Chymotrypsinogen in Chymotrypsin sowie bei den Kallikrein-Kinin-Kaskaden und der Blutgerinnungskaskade. Je nach Beschaffenheit des reaktiven Zentrums der daran beteiligten Proteinasen werden diese in die Klassen der Serin-Proteinasen (z.B. Chymotrypsin, Trypsin, Elastase und Kathepsin G), der Aspartat-Proteinasen (z.B. Kathepsin D, Kathepsin E und Pepsin), der Cystein-Proteinasen (z.B. Kathepsin B, Kathepsin H und Kathepsin L) und der Metallo-Proteinasen (z.B. Kollagenase und Thermolysin) unterteilt.

Um die oft kaskadenartig verlaufenden proteolytischen Prozesse gegenregulieren zu können, verfügt der Organismus über eine Reihe von anderen Proteinen, den Proteinase-Inhibitoren (zur Übersicht siehe Laskowski und Kato, 1980 und Bode und Huber, 1992). So schützen die in der Leber synthetisierten, humanen Plasma-Proteinase-Inhibitoren α₁-Antichymotrypsin und α₁-Proteinase-Inhibitoren das Lungengewebe vor unspezifischem Angriff durch die Proteinasen Kathepsin G bzw. Elastase aus polymorphkernigen Lymphozyten. Bei einem Ungleichgewicht zwischen Proteinasen und ihren spezifischen Inhibitoren kann es zum Auftreten pathologischer Effekte kommen. Ein übermäßiges Verhältnis von Elastase zu α₁-Proteinase-Inhibitor erhöht beispielsweise bei Patienten mit genetisch bedingtem Mangel an diesem Faktor das Risiko der Bildung eines Lungenemphysems um ca. 20 bis 30fach gegenüber der Normalbevölkerung (Carrel und Owen, 1980). Bei Rauchern wird die Emphysembildung mittels Oxidation der im reaktiven Zentrum des α₁-Proteinase-Inhibitors befindlichen Aminosäure Methionin durch im Zigarettenrauch enthaltene Oxidantien begünstigt (Miller und Kuschner, 1969; Ohlsson et al., 1980). Auch im Falle der Infektion mit Gram-negativen Bakterien können deren Endotoxine eine Desintegration von Phagozyten und damit die Ausschüttung lysosomaler Proteinasen verursachen, was durch den erhöhten Verbrauch an Proteinase-Inhibitoren unkontrollierte Gewebsschädigung und Entzündungen verursachen kann. Aus diesem Grund besitzen bestimmte Proteinase-Inhibitoren ein hohes therapeutisches Potential (siehe z.B. Fritz, 1980).

Das der Erfindung zugrunde liegende technische Problem besteht darin, weitere Inhibitoren von Serin-Proteinasen zur Verfügung zu stellen. Des weiteren sollten die für die erfindungsgemäßen Inhibitoren kodierenden Gene bzw. cDNA zur Verfügung gestellt werden.

Spezifisches Merkmal, der erfindungsgemäßen Serin-Proteinase-Inhibitoren ist, dass der Serin-Proteinase-Inhibitor eine Domäne mit vier Cysteinen aufweist und die Sequenz zwischen dem ersten und zweiten Cystein ausgewählt wird aus

die Sequenz zwischen dem zweiten und dritten Cystein ausgewählt wird aus und die Sequenz zwischen dem dritten und vierten Cystein ausgewählt wird aus AT, AL, AM, SM oder TM.

Besonders bevorzugt wird, dass der erfindungsgemäße Serin-Proteinase-Inhibitor einer der Formeln

R₁-C-HEFQAFMKNGKLF-C-PQDKKFFQSLDGIMFINK-C-AT-C-R₂

R₁-C-DDFKKGERDGDFI-C-PDYYEAVCGTDGKTYDNR-C-AL-C-R₂

R₁-C-SAFRPFVRNGRLG-C-TRENDPVLGPDGKTHGNK-C-AM-C-R₂

R₁-C-KEYEKQVRNGRLF-C-TRESDPVRGPDGRMHGNK-C-AL-C-R₂

R₁-C-SQYQNQAKNGILF-C-TRENDPIRGPDGKMHGNL-C-SM-C-R₂

R₁-C-NEYRKLVRNGKLA-C-TRENDPIQGPDGKVHGNT-C-SM-C-R₂

R₁-C-SEYRKSRKNGRLF-C-TRENDPIQGPDGKMHGNT-C-SM-C-R₂

R₁-C-SEFRDQVRNGTLI-C-TREHNPVRGPDGKMHGNK-C-AM-C-R₂

R₁-C-SEYRHYVRNGRLP-C-TRENDPIEGLDGKIHGNT-C-SM-C-R₂

R₁-C-DEFRRLLQNGKLF-C-TRENDPVRGPDGKTHGNK-C-AM-C-R₂

R₁-C-AEYREQMKNGRLS-C-TRESDPVRDADGKSYNNQ-C-TM-C-R₂

R₁-C-DEFRSQMKNGKLI-C-TRESDPVRGPDGKTHGNK-C-TM-C-R₂,

worin R₁ NH₂, eine Aminosäure oder ein Peptid mit bis zu 100 Aminosäuren ist und R₂ COOH, CONH₂, eine Aminosäure oder ein Peptid mit bis zu 100 Aminosäuren ist, entspricht.

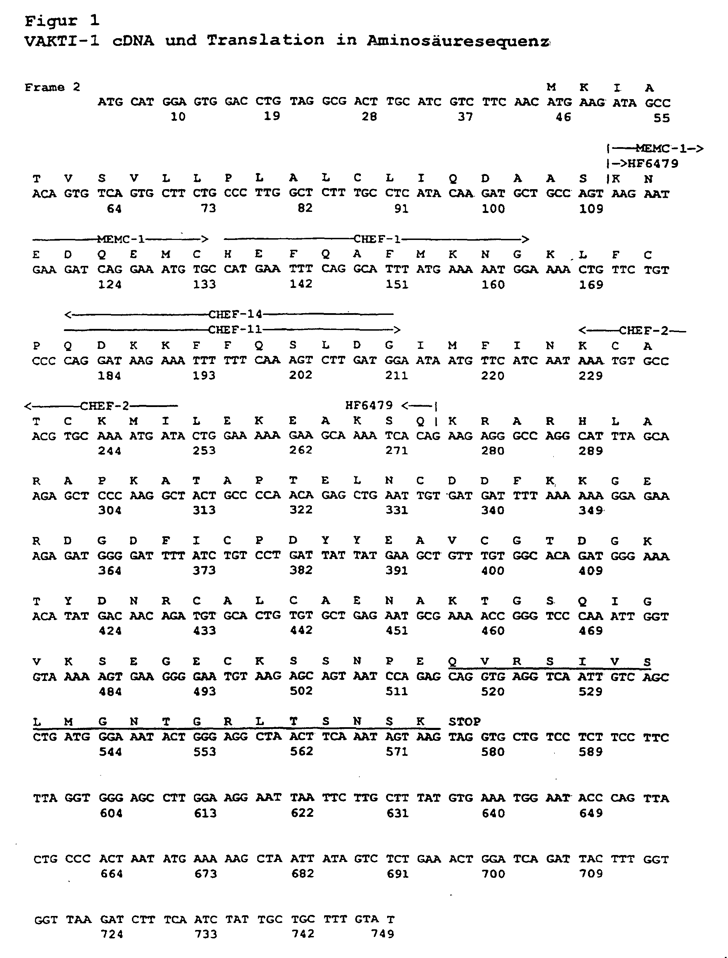

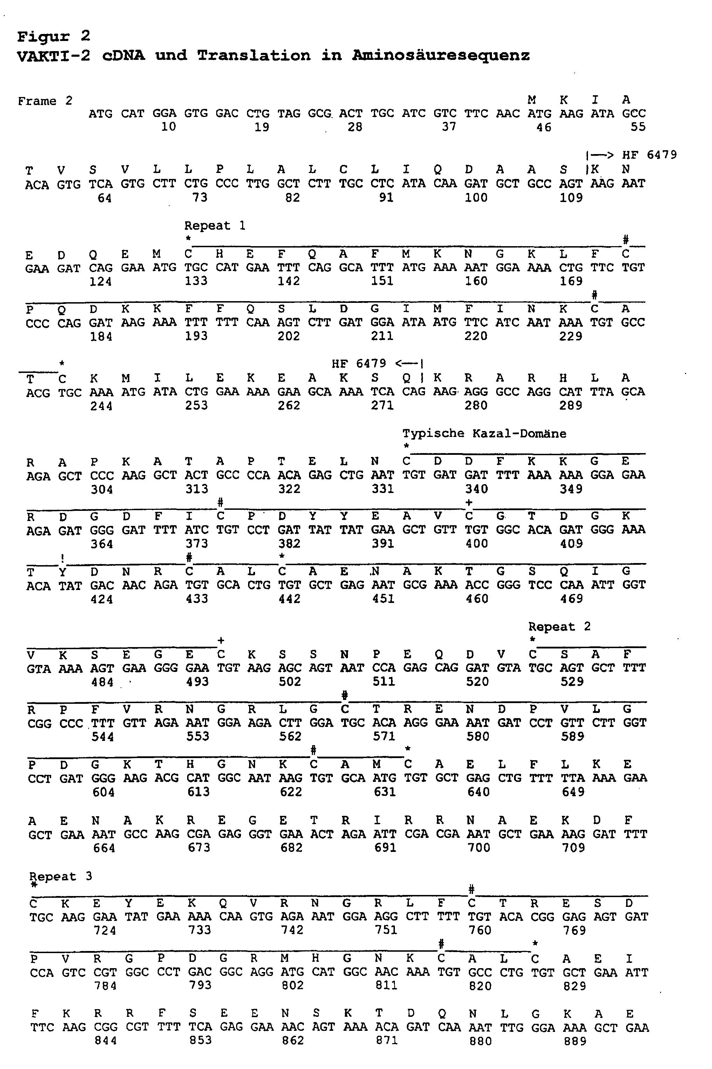

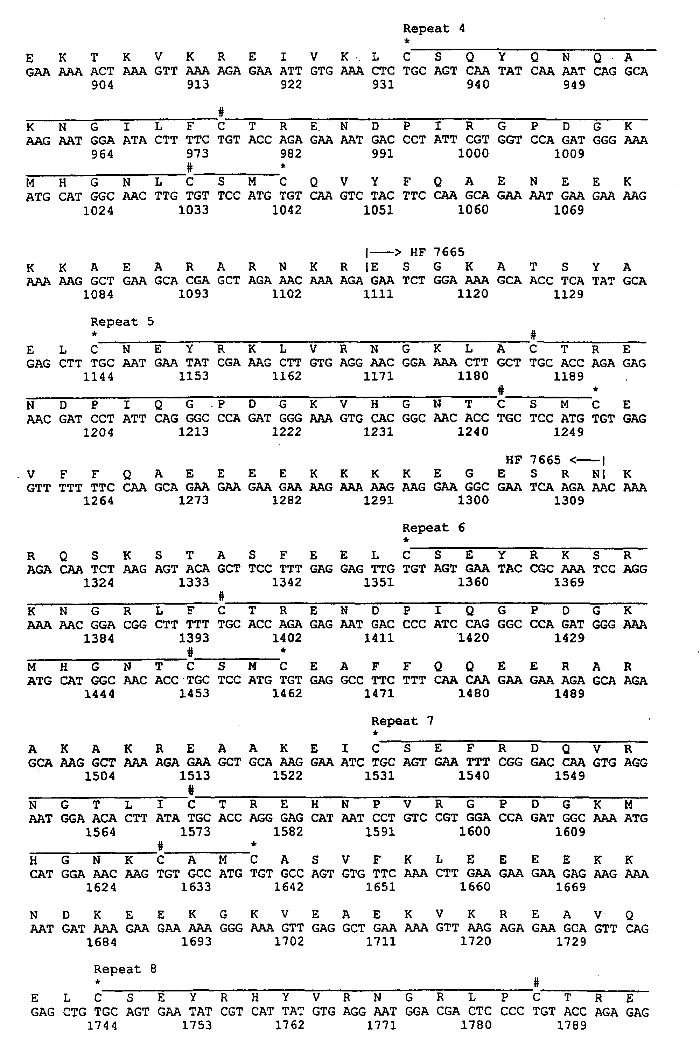

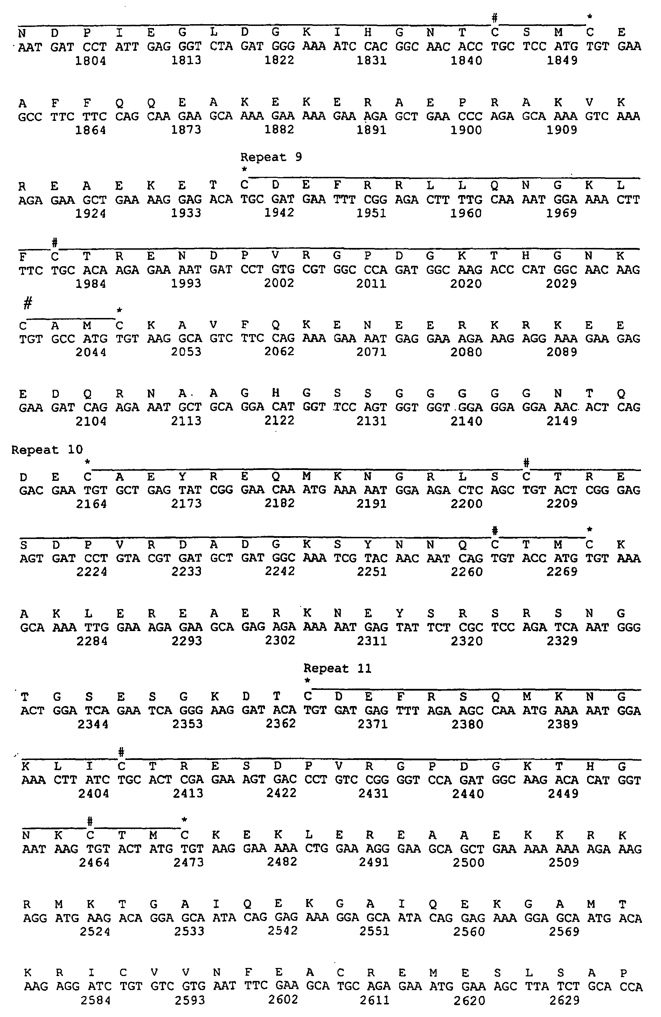

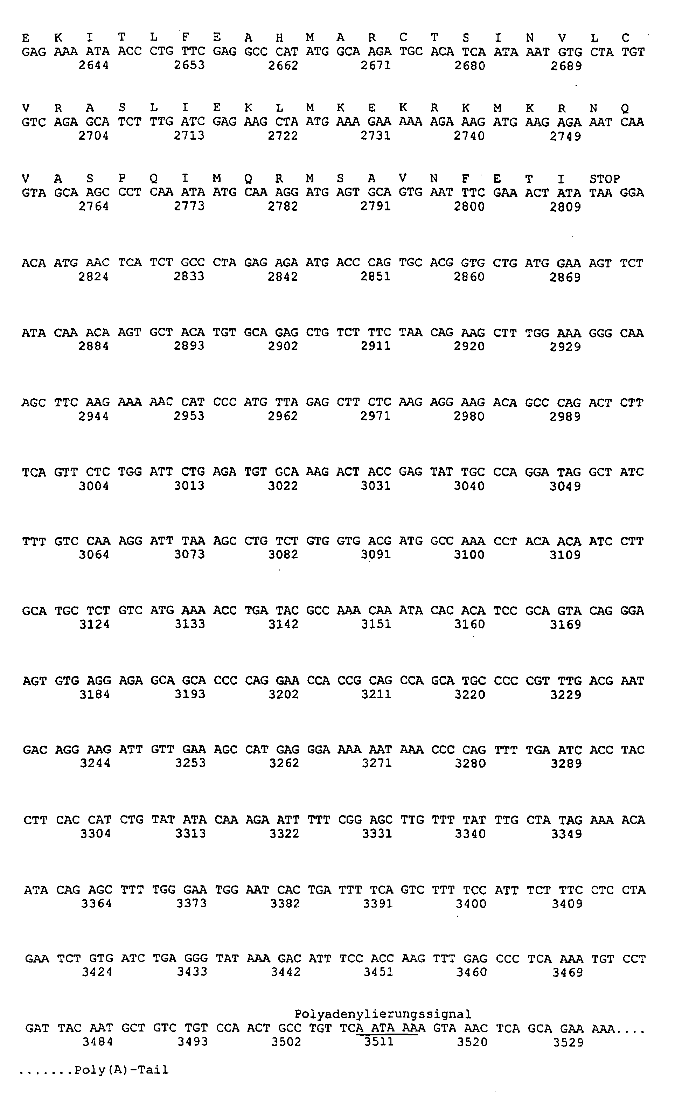

Bevorzugte Vertreter der erfindungsgemäßen Serin-Proteinase-Inhibitoren sind die Verbindungen HF 6479 und HF 7665 sowie Fragmente der Proteine VAKTI-1 und VAKTI-2 gemäß den Figuren 1 und 2.

Aus den Figuren 1 bis 3 lassen sich neben der Aminosäuresequenz der erfindungsgemäß bevorzugten Verbindungen auch weitere Informationen bezüglich der cDNA, die für die erfindungsgemäßen Verbindungen kodiert, entnehmen. Insbesondere werden die entsprechenden Motive und Primer hybridisierenden Stellen angegeben.

Die erfindungsgemäße Verbindung HF 3479 weist eine Masse von 6.479 Dalton auf, diejenige von HF 7665 beträgt 7.665 Dalton, beide wurden aus Hämofiltrat aufgereinigt.

Erfindungsgemäß beansprucht wird auch eine cDNA, kodierend für die erfindungsgemäßen Verbindungen, insbesondere eine cDNA mit der Nucleinsäuresequenz gemäß Figuren 1 bis 2.

Die erfindungsgemäßen Verbindungen sind als Arzneimittel geeignet. Gegebenenfalls werden sie zusammen mit pharmazeutisch verträglichen Trägerstoffen appliziert.

Die erfindungsgemäßen Arzneimittel enthaltend die erfindungsgemäßen Proteinase-Inhibitoren werden vorzugsweise in Mengen von 1 bis 100 mg/kg Körpergewicht des Patienten verabreicht. Als Verabreichungsform kommen alle galenischen Zubereitungen für Peptidwirkstoffe in Frage. Die Arzneimittel enthaltend Nucleinsäuren gemäß der Erfindung werden vorzugsweise in Mengen von 0,1 bis 100 mg/kg Körpergewicht eines entsprechen den Patienten verabreicht. Hier kommen als galenische Verabreichungsformen solche in Betracht, die zur Applikation von Nucleinsäuren geeignet sind, ohne dass die Nucleinsäuren vor Erreichen des Wirkortes durch Stoffwechseleinflüsse unwirksam gemacht werden. Als galenische Verabreichungsform können z.B. Liposomen eingesetzt werden, in denen die Nucleinsäuren befindlich sind.

Die erfindungsgemäßen Polypeptide, insbesondere VAKTI-I und VAKTI-II, können zur Herstellung von Antikörpern oder Antikörperfragmenten dienen. Diese werden in einfacher Weise durch Immunisierung geeigneter Säuger hergestellt. Durch an sich bekannte Operationen können die Antikörper auch humanisiert werden.

Die erfindungsgemäßen Verbindungen sind in einfacher Weise durch an sich bekannte Methoden der Peptid- bzw. Nucleotidsynthese herstellbar. Einer gentechnischen Herstellung der Verbindungen steht ebenfalls nichts im Wege.

Der Fachmann erkennt, daß bei den Polypeptiden gemäß der Erfindung auch Fragmente verwendet werden können, sofern sie die inhibitorischen Eigenschaften der Serin-Proteinase-Inhibitoren beibehalten. Das Auffinden solcher Fragmente ist dem Fachmann bekannt. So erfolgt dies beispielsweise durch gezielte enzymatische Spaltung der erfindungsgemäßen Verbindungen. Es können auch in den Seitenketten modifizierte Aminosäuren eingesetzt werden. Auch N- und C-terminal modifizierte Polypeptide kommen in Betracht. Insbesondere können phosphorylierte, glycosylierte, methylierte, acetylierte oder in ähnlicher Weise modifizierte Polypeptide eingesetzt werden, sofern sie die Wirkung der Serin-Proteinase-Inhibitoren nicht relevant beeinträchtigen.

Bei den Nucleinsäuren gemäß der Erfindung kommen auch Derivate in Betracht, die je nach Codon Usage modifizierte Tripletstrukturen aufweisen. Desweiteren sind als Nucleinsäuren gemäß der Erfindung auch solche zu verstehen, die durch Nucleasen gegenüber den nativen Verbindungen weniger stark abgebaut werden, beispielsweise die entsprechenden SODN-Derivate, die in der Antisense-Technologie üblicherweise eingesetzt werden, um die Antisense-Strukturen gegenüber enzymatischen Angriffen stabiler auszugestalten.

Auch mit den Polypeptiden homologe Strukturen kommen in Betracht. Dies sind insbesondere Polypeptidstrukturen, bei denen Aminosäuren ausgetauscht sind. So können beispielsweise konservative Aminosäuresubstitutionen in hochkonservierten Regionen wie folgt berücksichtigt werden: Jede Isoleucin-, Valin- und Leucin-Aminosäure kann gegen eine andere dieser Aminosäuren ausgetauscht sein, Aspartat kann gegen Glutamat und umgekehrt ausgetauscht sein, Glutamin gegen Asparagin und umgekehrt, Serin gegen Trionin und umgekehrt. Konservative Aminosäuresubstitutionen in weniger hochkonservierten Regionen können wie folgt sein: Jede der Aminosäuren Isoleucin, Valin und Leucin gegen jede andere Aminosäuren, Aspartat gegen Glutamat und umgekehrt, Glutamin gegen Asparagin und umgekehrt, Serin gegen Theonin und umgekehrt, Glycin gegen Alanin und umgekehrt, Alanin gegen Valin und umgekehrt, Methionin gegen jede der Aminosäuren Leucin, Isoleucin oder Valin, Lysin gegen Arginin und umgekehrt, eine der Aminosäuren Aspartat oder Glutamat gegen eine der Aminosäuren Arginin oder Lysin, Histidin gegen eine der Aminosäuren Arginin oder Lysin, Glutamin gegen Glutamat und umgekehrt und Asparagin gegen Aspartat und umgekehrt.

Die Wirkungsweise der erfindungsgemäßen Peptide wird durch das folgende Beispiel erläutert.

### Beispiel

Messung der Proteinase Inhibition durch HF 7665

### Meßansatz:

| | |
|---|---|
| 84 µl | Meßpuffer (0,1 M HEPES, pH 7.5; 0,5 M NaCl₂) |
| 1 µl | Trypsin (1 mg/ml in 1 mM HCl, 20 mM CaCl₂) |
| 5 µl | L-BABNA (6 mg/ml Na-Benzoyl-L-Arginine-p-Nitro- |
| | anilide Hydrochloride) |
| 10 µl | Proteinase-Inhibitor (10 µM bzw. 75 µg/ml HF 7665 |
| | in H₂O). |

Die Reaktion wurde durch Zugabe des chromogenen Substraten gestartet und der Substratumsatz mittels Photometer bei λ-405 nm verfolgt. Nach ca. fünf Minuten wurden 10 µl Prc teinase-Inhibitor bzw. entsprechende Kontrollen dazugegeben und der weitere Extinktionsverlauf beobachtet.

Es konnte gezeigt werden, daß HF 7665 in einer Endkonzentration von ca. 1 µM bzw. 7,5 µg/ml einen inhibitorischen Effekt auf Trypsin besitzt. Kontrollversuche mit entsprechenden Mengen an BSA (7,5 µg/ml) und Acetonitril/TFA (0,8% ACN/0,001% TFA) zeigten keine Trypsininhibierung. Weiterhin konnte kein inhibitorischer Effekt von HF 7665 auf Chymotrypsin bei einem ähnlichen Test beobachtet werden.

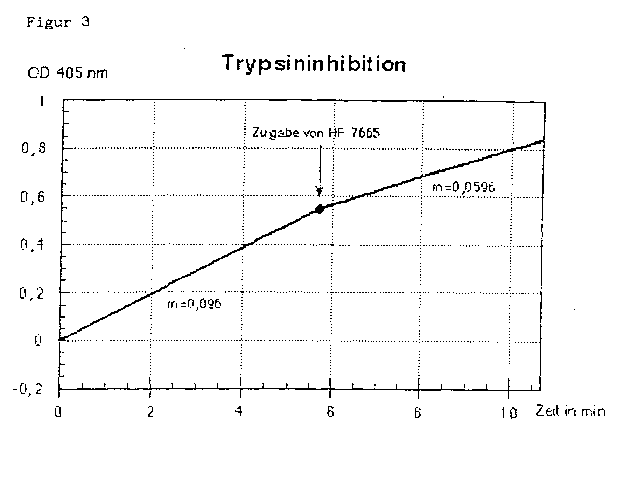

Figur 3 zeigt, daß sich nach Zugabe von HF 7665 der Substratumsatz durch Trypsininhibierung um ca. 30% vermindert.

## Patentansprüche

1. Serin-Proteinase-Inhibitor, **dadurch gekennzeichnet, dass** die Sequenz der Domäne zwischen dem ersten und dem zweiten Cystein ausgewählt wird aus die Sequenz zwischen dem zweiten und dritten Cystein der Domäne ausgewählt wird aus und die Sequenz zwischen dem dritten und vierten Cystein der Domäne ausgewählt wird aus
AT, AL, AM, SM oder TM.

2. Serin-Proteinase-Inhibitor gemäß Anspruch 1, mit einer der Formeln
R₁-C-HEFQAFMKNGKLF-C-PQDKKFFQSLDGIMFINK-C-AT-C-R₂
R₁-C-DDFKKGERDGDFI-C-PDYYEAVCGTDGKTYDNR-C-AL-C-R₂
R₁-C-SAFRPFVRNGRLG-C-TRENDPVLGPDGKTHGNK-C-AM-C-R₂
R₁-C-KEYEKQVRNGRLF-C-TRESDPVRGPDGRMHGNK-C-AL-C-R₂
R₁-C-SQYQNQAKNGILF-C-TRENDPIRGPDGKMHGNL-C-SM-C-R₂
R₁-C-NEYRKLVRNGKLA-C-TRENDPIQGPDGKVHGNT-C-SM-C-R₂
R₁-C-SEYRKSRKNGRLF-C-TRENDPIQGPDGKMHGNT-C-SM-C-R₂
R₁-C-SEFRDQVRNGTLI-C-TREHNPVRGPDGKMHGNK-C-AM-C-R₂
R₁-C-SEYRHYVRNGRLP-C-TRENDPIEGLDGKIHGNT-C-SM-C-R₂
R₁-C-DEFRRLLQNGKLF-C-TREN DPVRGPDGKTHGNK-C-AM-C-R₂
R₁-C-AEYREQMKNGRLS-C-TRESDPVRDADGKSYNNQ-C-TM-C-R₂
R₁-C-DEFRSQMKNGKLI-C-TRESDPVRGPDGKTHGNK-C-TM-C-R₂,
worin R₁ NH₂, eine Aminosäure oder ein Peptid mit bis zu 1.000 Aminosäuren ist und R₂ COOH, CONH₂, eine Aminosäure oder ein Peptid mit bis zu 1.000 Aminosäuren ist.

3. Serin-Proteinase-Inhibitor gemäß mindestens einem der Ansprüche 1 und/oder 2, **dadurch gekennzeichnet, dass** es sich um ein Fragment von VAKTI-1 (Seq. ID Nr.1) oder VAKTI-2 (Seq. ID Nr.2) handelt.

4. Serin-Proteinase-Inhibitor gemäß Anspruch 3, **dadurch gekennzeichnet, dass** es sich um HF 6479 (Seq. ID Nr.3) oder HF 7665 (Seq. ID Nr.4) handelt.

5. Nucleinsäure, kodierend für einen Serin-Proteinase-Inhibitor gemäß mindestens einem der Ansprüche 1 bis 4.

6. Arzneimittel enthaltend mindestens einen Serin-Proteinase-Inhibitor gemäß mindestens einem der Ansprüche 1 bis 4 und/oder eine Nucleinsäure gemäß Anspruch 5 gegebenenfalls mit pharmazeutischen Trägerstoffen.

7. Arzneimittel nach Anspruch 6, enthaltend 0,01 bis 1.000 mg/kg Körpergewicht des Serin-Proteinase-Inhibitors nach mindestens einem der Ansprüche 1 bis 4 und/oder der Nucleinsäure gemäß Anspruch 5.

8. Antikörper oder Antikörper-Fragmente gegen Epitope der Verbindungen nach einem der Ansprüche 1 bis 4.

9. Poly- oder Oligonucleotide, die mit Bereichen der cDNA oder entsprechender RNA unter stringenten Bedingungen hybridisieren und die Expression codierender Bereiche der für die Verbindungen nach Ansprüchen 1 bis 4 codierenden Gene verhindern (Antisense Verbindungen).

10. DNA kodierend für die in den Ansprüchen 1 bis 4 genannten Verbindungen.

11. DNA gemäß Anspruch 10 mit der Seq. ID Nr. 5 oder Seq. ID Nr. 6.

## Claims

1. A serine protease inhibitor, **characterized in that** the sequence of the domain between the first and second cysteines is selected from the sequence between the second and third cysteines of the domain is selected from the sequence between the third and fourth cysteines of the domain is selected from
AT, AL, AM, SM, or TM.

2. The serine protease inhibitor according to claim 1, having one of the following formulas:
R₁-C-HEFQAFMKNGKLF-C-PQDKKFFQSLDGIMFINK-C-AT-C-R₂
R₁-C-DDFKKGERDGDFI-C-PDYYEAVCGTDGKTYDNR-C-AL-C-R₂
R₁-C-SAFRPFVRNGRLG-C-TRENDPVLGPDGKTHGNK-C-AM-C-R₂
R₁-C-KEYEKQVRNGRLF-C-TRESDPVRGPDGRMHGNK-C-AL-C-R₂
R₁-C-SQYQNQAKNGILF-C-TRENDPIRGPDGKMHGNL-C-SM-C-R₂
R₁-C-NEYRKLVRNGKLA-C-TRENDPIQGPDGKVHGNT-C-SM-C-R₂
R₁-C-SEYRKSRKNGRLF-C-TRENDPIQGPDGKMHGNT-C-SM-C-R₂
R₁-C-SEFRDQVRNGTLI-C-TREHNPVRGPDGKMHGNK-C-AM-C-R₂
R₁-C-SEYRHYVRNGRLP-C-TRENDPIEGLDGKIHGNT-C-SM-C-R₂
R₁-C-DEFRRLLQNGKLF-C-TRENDPVRGPDGKTHGNK-C-AM-C-R₂
R₁-C-AEYREQMKNGRLS-C-TRESDPVRDADGKSYNNQ-C-TM-C-R₂
R₁-C-DEFRSQMKNGKLI-C-TRESDPVRGPDGKTHGNK-C-TM-C-R₂,
wherein R₁ is NH₂, an amino acid, or a peptide with up to 1000 amino acids, and R₂ is COOH, CONH₂, an amino acid, or a peptide with up to 1000 amino acids.

3. The serine protease inhibitor according to at least one of claims 1 and/or 2, **characterized by** being a fragment of VAKTI-1 (SEQ. ID. NO. 1) or VAKTI-2 (SEQ. ID. NO. 2).

4. The serine protease inhibitor according to claim 3, **characterized by** being HF 6479 (SEQ. ID. NO. 3) or HF 7665 (SEQ. ID. NO. 4).

5. A nucleic acid coding for a serine protease inhibitor according to at least one of claims 1 to 4.

6. A medicament containing at least one serine protease inhibitor according to at least one of claims 1 to 4 and/or a nucleic acid according to claim 5, optionally together with pharmaceutical vehicles.

7. The medicament according to claim 6, containing from 0.01 to 1000 mg per kg of body weight of the serine protease inhibitor according to at least one of claims 1 to 4 and/or of the nucleic acid according to claim 5.

8. Antibodies or antibody fragments against epitopes of the compounds according to any of claims 1 to 4.

9. Poly- or oligonucleotides which will hybridize to regions of the cDNA or corresponding RNA under stringent conditions and prevent the expression of coding regions of the genes coding for the compounds according to claims 1 to 4 (antisense compounds).

10. DNA, coding for the compounds mentioned in claims 1 to 4.

11. The DNA according to claim 10 having the SEQ. ID. NO. 5 or SEQ. ID. NO. 6.

## Revendications

1. Inhibiteur de sérine-protéinase, **caractérisé en ce que** la séquence du domaine entre la première et la seconde cystéine est sélectionnée parmi le groupe constitué des séquences suivantes la séquence du domaine situé entre la seconde et la troisième cystéine est sélectionnée parmi le groupe constitué des séquences suivantes et la séquence du domaine situé entre la troisième et la quatrième cystéine est sélectionnée parmi le groupe constitué des séquences suivantes
AT, AL, AM, SM ou TM.

2. Inhibiteur de sérine-protéinase selon la revendication 1, ayant une des formules suivantes :
R₁-C-HEFQAFMKNGKLF-C-PQDKKFFQSLDGIMFINK-C-AT-C-R₂
R₁-C-DDFKKGERDGDFI-C-PDYYEAVCGTDGKTYDNR-C-AL-C-R₂
R₁-C-SAFRPFVRNGRLG-C-TRENDPVLGPDGKTHGNK-C-AM-C-R₂
R₁-C-KEYEKQVRNGRLF-C-TRESDPVRGPDGRMHGNK-C-AL-C-R₂
R₁-C-SQYQNQAKNGILF-C-TRENDPIRGPDGKMHGNL-C-SM-C-R₂
R₁-C-NEYRKLVRNGKLA-C-TRENDPIQGPDGKVHGNT-C-SM-C-R₂
R₁-C-SEYRKSRKNGRLF-C-TRENDPIQGPDGKMHGNT-C-SM-C-R₂
R₁-C-SEFRDQVRNGTLI-C-TREHNPVRGPDGKMHGNK-C-AM-C-R₂
R₁-C-SEYRHYVRNGRLP-C-TRENDPIEGLDGKIHGNT-C-SM-C-R₂
R₁-C-DEFRRLLQNGKLF-C-TRENDPVRGPDGKTHGNK-C-AM-C-R₂
R₁-C-AEYREQMKNGRLS-C-TRESDPVRDADGKSYNNQ-C-TM-C-R₂
R₁-C-DEFRSQMKNGKLI-C-TRESDPVRGPDGKTHGNK-C-TM-C-R₂,
dans lesquelles R₁ est NH₂, un acide aminé ou un peptide comprenant jusqu'à 1000 acides aminés et R₂ est COOH, CONH₂, un acide aminé ou un peptide comprenant jusqu'à 1000 acides aminés.

3. Inhibiteur de sérine-protéinase selon au moins une des revendications 1 et/ou 2, **caractérisé en ce qu'**il s'agit d'un fragment de VAKTI-1 (Seq. ID N° 1) ou de VAKTI-2 (Seq. ID N° 2).

4. Inhibiteur de sérine-protéinase selon la revendication 3, **caractérisé en ce qu'**il s'agit d'HF 6479 (Seq. ID N° 3) ou d'HF 7665 (Seq. ID N° 4).

5. Acide nucléique codant pour un inhibiteur de sérine-protéinase selon au moins une des revendications 1 à 4.

6. Médicament contenant au moins un inhibiteur de sérine-protéinase selon au moins une des revendications 1 à 4 et/ou un acide nucléique selon la revendication 5 éventuellement avec des substances de support pharmaceutiques.

7. Médicament selon la revendication 6, contenant 0,01 à 1.000 mg/kg de poids corporel d'inhibiteur de sérine-protéinase selon au moins une des revendications 1 à 4 et/ou d'acide nucléique selon la revendication 5.

8. Anticorps ou fragment d'anticorps de l'épitope des composés selon une des revendications 1 à 4.

9. Poly- ou oligonucléotides, qui hybrident dans les domaines de l'ADNc ou de l'ARN correspondant dans des conditions stringentes et qui empêchent l'expression des domaines codant des gènes codant pour les composés selon les revendications 1 à 4 (composés antisens).

10. ADN codant pour les composés mentionnés dans les revendications 1 à 4.

11. ADN selon la revendication 10 ayant la séquence Seq. ID N° 5 ou Seq. ID N° 6.


### SEQUENZPROTOKOLL

(1) ALLGEMEINE ANGABEN:
   (i) ANMELDER:
      (A) NAME: Prof. Dr. Wolf-Georg Forssmann
      (B) STRASSE: Feodor-Lynen-Str. 31
      (C) ORT: Hannover
      (E) LAND: Deutschland
      (F) POSTLEITZAHL: 30625
   (ii) BEZEICHNUNG DER ERFINDUNG: Serin-Proteinase-Inhibitoren
   (iii) ANZAHL DER SEQUENZEN: 34
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 177 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 1:
   Met Lys Ile Ala Thr Val Ser Val Leu Leu Pro Leu Ala Leu Cys Leu
   1               5                   10                  15
   Ile Gln Asp Ala Ala Ser Lys Asn Glu Asp Gln Glu Met Cys His Glu
               20                  25                  30
   Phe Gln Ala Phe Met Lys Asn Gly Lys Leu Phe Cys Pro Gln Asp Lys
           35                  40                  45
   Lys Phe Phe Gln Ser Leu Asp Gly Ile Met Phe Ile Asn Lys Cys Ala
       50                  55                  60
   Thr Cys Lys Met Ile Leu Glu Lys Glu Ala Lys Ser Gln Lys Arg Ala
   65                  70                  75                  80
   Arg His Leu Ala Arg Ala Pro Lys Ala Thr Ala Pro Thr Glu Leu Asn
                   85                  90                  95
   Cys Asp Asp Phe Lys Lys Gly Glu Arg Asp Gly Asp Phe Ile Cys Pro
               100                 105                 110
   Asp Tyr Tyr Glu Ala Val Cys Gly Thr Asp Gly Lys Thr Tyr Asp Asn
           115                 120                 125
   Arg Cys Ala Leu Cys Ala Glu Asn Ala Lys Thr Gly Ser Gln Ile Gly
       130                 135                 140
   Val Lys Ser Glu Gly Glu Cys Lys Ser Ser Asn Pro Glu Gln Val Arg
   145                 150                 155                 160
   Ser Ile Val Ser Leu Met Gly Asn Thr Gly Arg Leu Thr Ser Asn Ser
                   165                 170                 175
   Lys
(2) ANGABEN ZU SEQ ID NO: 2:
   (i) SEQUENZKENNZEICHEN:
      (A) LÄNGE: 922 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 2:
   Met Lys Ile Ala Thr Val Ser Val Leu Leu Pro Leu Ala Leu Cys Leu
   1               5                   10                  15
   Ile Gln Asp Ala Ala Ser Lys Asn Glu Asp Gln Glu Met Cys His Glu
               20                  25                  30
   Phe Gln Ala Phe Met Lys Asn Gly Lys Leu Phe Cys Pro Gln Asp Lys
           35                  40                  45
   Lys Phe Phe Gln Ser Leu Asp Gly Ile Met Phe Ile Asn Lys Cys Ala
       50                  55                  60
   Thr Cys Lys Met Ile Leu Glu Lys Glu Ala Lys Ser Gln Lys Arg Ala
   65                  70                  75                  80
   Arg His Leu Ala Arg Ala Pro Lys Ala Thr Ala Pro Thr Glu Leu Asn
                   85                  90                  95
   Cys Asp Asp Phe Lys Lys Gly Glu Arg Asp Gly Asp Phe Ile Cys Pro
               100                 105                 110
   Asp Tyr Tyr Glu Ala Val Cys Gly Thr Asp Gly Lys Thr Tyr Asp Asn
           115                 120                 125
   Arg Cys Ala Leu Cys Ala Glu Asn Ala Lys Thr Gly Ser Gln Ile Gly
       130                 135                 140
   Val Lys Ser Glu Gly Glu Cys Lys Ser Ser Asn Pro Glu Gln Asp Val
   145                 150                 155                 160
   Cys Ser Ala Phe Arg Pro Phe Val Arg Asn Gly Arg Leu Gly Cys Thr
                   165                 170                 175
   Arg Glu Asn Asp Pro Val Leu Gly Pro Asp Gly Lys Thr His Gly Asn
               180                 185                 190
   Lys Cys Ala Met Cys Ala Glu Leu Phe Leu Lys Glu Ala Glu Asn Ala
           195                 200                 205
   Lys Arg Glu Gly Glu Thr Arg Ile Arg Arg Asn Ala Glu Lys Asp Phe
       210                 215                 220
   Cys Lys Glu Tyr Glu Lys Gln Val Arg Asn Gly Arg Leu Phe Cys Thr
   225                 230                 235                 240
   Arg Glu Ser Asp Pro Val Arg Gly Pro Asp Gly Arg Met His Gly Asn
                   245                 250                 255
   Lys Cys Ala Leu Cys Ala Glu Ile Phe Lys Arg Arg Phe Ser Glu Glu
               260                 265                 270
   Asn Ser Lys Thr Asp Gln Asn Leu Gly Lys Ala Glu Glu Lys Thr Lys
           275                 280                 285
   Val Lys Arg Glu Ile Val Lys Leu Cys Ser Gln Tyr Gln Asn Gln Ala
       290                 295                 300
   Lys Asn Gly Ile Leu Phe Cys Thr Arg Glu Asn Asp Pro Ile Arg Gly
   305                 310                 315                 320
   Pro Asp Gly Lys Met His Gly Asn Leu Cys Ser Met Cys Gln Val Tyr
                   325                 330                 335
   Phe Gln Ala Glu Asn Glu Glu Lys Lys Lys Ala Glu Ala Arg Ala Arg
               340                 345                 350
   Asn Lys Arg Glu Ser Gly Lys Ala Thr Ser Tyr Ala Glu Leu Cys Asn
           355                 360                 365
   Glu Tyr Arg Lys Leu Val Arg Asn Gly Lys Leu Ala Cys Thr Arg Glu
       370                 375                 380
   Asn Asp Pro Ile Gln Gly Pro Asp Gly Lys Val His Gly Asn Thr Cys
   385                 390                 395                 400
   Ser Met Cys Glu Val Phe Phe Gln Ala Glu Glu Glu Glu Lys Lys Lys
                   405                 410                 415
   Lys Glu Gly Glu Ser Arg Asn Lys Arg Gln Ser Lys Ser Thr Ala Ser
               420                 425                 430
   Phe Glu Glu Leu Cys Ser Glu Tyr Arg Lys Ser Arg Lys Asn Gly Arg
           435                 440                 445
   Leu Phe Cys Thr Arg Glu Asn Asp Pro Ile Gln Gly Pro Asp Gly Lys
       450                 455                 460
   Met His Gly Asn Thr Cys Ser Met Cys Glu Ala Phe Phe Gln Gln Glu
   465                 470                 475                 480
   Glu Arg Ala Arg Ala Lys Ala Lys Arg Glu Ala Ala Lys Glu Ile Cys
                   485                 490                 495
   Ser Glu Phe Arg Asp Gln Val Arg Asn Gly Thr Leu Ile Cys Thr Arg
               500                 505                 510
   Glu His Asn Pro Val Arg Gly Pro Asp Gly Lys Met His Gly Asn Lys
           515                 520                 525
   Cys Ala Met Cys Ala Ser Val Phe Lys Leu Glu Glu Glu Glu Lys Lys
       530                 535                 540
   Asn Asp Lys Glu Glu Lys Gly Lys Val Glu Ala Glu Lys Val Lys Arg
   545                 550                 555                 560
   Glu Ala Val Gln Glu Leu Cys Ser Glu Tyr Arg His Tyr Val Arg Asn
                   565                 570                 575
   Gly Arg Leu Pro Cys Thr Arg Glu Asn Asp Pro Ile Glu Gly Leu Asp
               580                 585                 590
   Gly Lys Ile His Gly Asn Thr Cys Ser Met Cys Glu Ala Phe Phe Gln
   595 600 605
   Gln Glu Ala Lys Glu Lys Glu Arg Ala Glu Pro Arg Ala Lys Val Lys
   610 615 620
   Arg Glu Ala Glu Lys Glu Thr Cys Asp Glu Phe Arg Arg Leu Leu Gln
   625 630 635 640
   Asn Gly Lys Leu Phe Cys Thr Arg Glu Asn Asp Pro Val Arg Gly Pro
   645 650 655
   Asp Gly Lys Thr His Gly Asn Lys Cys Ala Met Cys Lys Ala Val Phe
   660 665 670
   Gln Lys Glu Asn Glu Glu Arg Lys Arg Lys Glu Glu Glu Asp Gln Arg
   675 680 685
   Asn Ala Ala Gly His Gly Ser Ser Gly Gly Gly Gly Gly Asn Thr Gln
   690 695 700
   Asp Glu Cys Ala Glu Tyr Arg Glu Gln Met Lys Asn Gly Arg Leu Ser
   705 710 715 720
   Cys Thr Arg Glu Ser Asp Pro Val Arg Asp Ala Asp Gly Lys Ser Tyr
   725 730 735
   Asn Asn Gln Cys Thr Met Cys Lys Ala Lys Leu Glu Arg Glu Ala Glu
   740 745 750
   Arg Lys Asn Glu Tyr Ser Arg Ser Arg Ser Asn Gly Thr Gly Ser Glu
   755 760 765
   Ser Gly Lys Asp Thr Cys Asp Glu Phe Arg Ser Gln Met Lys Asn Gly
   770 775 780
   Lys Leu Ile Cys Thr Arg Glu Ser Asp Pro Val Arg Gly Pro Asp Gly
   785 790 795 800
   Lys Thr His Gly Asn Lys Cys Thr Met Cys Lys Glu Lys Leu Glu Arg
   805 810 815
   Glu Ala Ala Glu Lys Lys Arg Lys Arg Met Lys Thr Gly Ala Ile Gln
   820 825 830
   Glu Lys Gly Ala Ile Gln Glu Lys Gly Ala Met Thr Lys Arg Ile Cys
   835 840 845
   Val Val Asn Phe Glu Ala Cys Arg Glu Met Glu Ser Leu Ser Ala Pro
   850 855 860
   Glu Lys Ile Thr Leu Phe Glu Ala His Met Ala Arg Cys Thr Ser Ile
   865 870 875 880
   Asn Val Leu Cys Val Arg Ala Ser Leu Ile Glu Lys Leu Met Lys Glu
   885 890 895
   Lys Arg Lys Met Lys Arg Asn Gln Val Ala Ser Pro Gln Ile Met Gln
   900 905 910
   Arg Met Ser Ala Val Asn Phe Glu Thr Ile
   915 920
(2) ANGABEN ZU SEQ ID NO: 3:
   (i) SEQUENZKENNZEICHEN:
      (A) LÄNGE: 55 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 3:
   Lys Asn Glu Asp Gln Glu Met Cys His Glu Phe Gln Ala Phe Met Lys
   1               5                   10                  15
   Asn Gly Lys Leu Phe Cys Pro Gln Asp Lys Lys Phe Phe Gln Ser Leu
               20                  25                  30
   Asp Gly Ile Met Phe Ile Asn Lys Cys Ala Thr Cys Lys Met Ile Leu
           35                  40                  45
   Glu Lys Glu Ala Lys Ser Gln
       50                  55
(2) ANGABEN ZU SEQ ID NO: 4:
   (i) SEQUENZKENNZEICHEN:
      (A) LÄNGE: 68 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 4:
   Glu Ser Gly Lys Ala Thr Ser Tyr Ala Glu Leu Cys Asn Glu Tyr Arg
   1               5                   10                  15
   Lys Leu Val Arg Asn Gly Lys Leu Ala Cys Thr Arg Glu Asn Asp Pro
               20                  25                  30
   Ile Gln Gly Pro Asp Gly Lys Val His Gly Asn Thr Cys Ser Met Cys
           35                  40                  45
   Glu Val Phe Phe Gln Ala Glu Glu Glu Glu Lys Lys Lys Lys Glu Gly
       50                  55                  60
   Glu Ser Arg Asn
   65
(2) ANGABEN ZU SEQ ID NO: 5:
   (i) SEQUENZKENNZEICHEN:
      (A) LÄNGE: 748 Basenpaare
      (B) ART: Nucleotid
      (C) STRANGFORM: Einzelstrang
      (D) TOPOLOGIE: nicht bekannt
   (ii) ART DES MOLEKÜLS: cDNA
   (xi) SEQUENZBESCHREIBUNG: SEQ ID NO: 5:
   ATGCATGGAG TGGACCTGTA GGCGACTTGC ATCGTCTTCA ACATGAAGAT AGCCACAGTG    60
   TCAGTGCTTC TGCCCTTGGC TCTTTGCCTC ATACAAGATG CTGCCAGTAA GAATGAAGAT   120
   CAGGAAATGT GCCATGAATT TCAGGCATTT ATGAAAAATG GAAAACTGTT CTGTCCCCAG   180
   GATAAGAAAT TTTTTCAAAG TCTTGATGGA ATAATGTTCA TCAATAAATG TGCCACGTGC   240
   AAAATGATAC TGGAAAAAGA AGCAAAATCA CAGAAGAGGG CCAGGCATTT AGCAAGAGCT   300
   CCCAAGGCTA CTGCCCCAAC AGAGCTGAAT TGTGATGATT TTAAAAAAGG AGAAAGAGAT   360
   GGGGATTTTA TCTGTCCTGA TTATTATGAA GCTGTTTGTG GCACAGATGG GAAAACATAT   420
   GACAACAGAT GTGCACTGTG TGCTGAGAAT GCGAAAACCG GGTCCCAAAT TGGTGTAAAA   480
   AGTGAAGGGG AATGTAAGAG CAGTAATCCA GAGCAGGTGA GGTCAATTGT CAGCCTGATG   540
   GGAAATACTG GGAGGCTAAC TTCAAATAGT AAGTAGGTGC TGTCCTCTTC CTTCTTAGGT   600
   GGGAGCCTTG GAAGGAATTA ATTCTTGCTT TATGTGAAAT GGAATACCCA GTTACTGCCC   660
   ACTAATATGA AAAAGCTAAT TATAGTCTCT GAAACTGGAT CAGATTACTT TGGTGGTTAA   720
   GATCTTTCAA TCTATTGCTG CTTTGTAT                                      748
(2) ANGABEN ZU SEQ ID NO: 6:
   (i) SEQUENZKENNZEICHEN:
      (A) LÄNGE: 3531 Basenpaare
      (B) ART: Nucleotid
      (C) STRANGFORM: Einzelstrang
      (D) TOPOLOGIE: nicht bekannt
   (ii) ART DES MOLEKÜLS: cDNA
   (xi) SEQUENZBESCHREIBUNG: SEQ ID NO: 6:
   ATGCATGGAG TGGACCTGTA GGCGACTTGC ATCGTCTTCA ACATGAAGAT AGCCACAGTG    60
   TCAGTGCTTC TGCCCTTGGC TCTTTGCCTC ATACAAGATG CTGCCAGTAA GAATGAAGAT   120
   CAGGAAATGT GCCATGAATT TCAGGCATTT ATGAAAAATG GAAAACTGTT CTGTCCCCAG   180
   GATAAGAAAT TTTTTCAAAG TCTTGATGGA ATAATGTTCA TCAATAAATG TGCCACGTGC   240
   AAAATGATAC TGGAAAAAGA AGCAAAATCA CAGAAGAGGG CCAGGCATTT AGCAAGAGCT   300
   CCCAAGGCTA CTGCCCCAAC AGAGCTGAAT TGTGATGATT TTAAAAAAGG AGAAAGAGAT   360
   GGGGATTTTA TCTGTCCTGA TTATTATGAA GCTGTTTGTG GCACAGATGG GAAAACATAT   420
   GACAACAGAT GTGCACTGTG TGCTGAGAAT GCGAAAACCG GGTCCCAAAT TGGTGTAAAA   480
   AGTGAAGGGG AATGTAAGAG CAGTAATCCA GAGCAGGATG TATGCAGTGC TTTTCGGCCC   540
   TTTGTTAGAA ATGGAAGACT TGGATGCACA AGGGAAAATG ATCCTGTTCT TGGTCCTGAT   600
   GGGAAGACGC ATGGCAATAA GTGTGCAATG TGTGCTGAGC TGTTTTTAAA AGAAGCTGAA   660
   AATGCCAAGC GAGAGGGTGA AACTAGAATT CGACGAAATG CTGAAAAGGA TTTTTGCAAG 720
   GAATATGAAA AACAAGTGAG AAATGGAAGG CTTTTTTGTA CACGGGAGAG TGATCCAGTC 780
   CGTGGCCCTG ACGGCAGGAT GCATGGCAAC AAATGTGCCC TGTGTGCTGA AATTTTCAAG 840
   CGGCGTTTTT CAGAGGAAAA CAGTAAAACA GATCAAAATT TGGGAAAAGC TGAAGAAAAA 900
   ACTAAAGTTA AAAGAGAAAT TGTGAAACTC TGCAGTCAAT ATCAAAATCA GGCAAAGAAT 960
   GGAATACTTT TCTGTACCAG AGAAAATGAC CCTATTCGTG GTCCAGATGG GAAAATGCAT 1020
   GGCAACTTGT GTTCCATGTG TCAAGTCTAC TTCCAAGCAG AAAATGAAGA AAAGAAAAAG 1080
   GCTGAAGCAC GAGCTAGAAA CAAAAGAGAA TCTGGAAAAG CAACCTCATA TGCAGAGCTT 1140
   TGCAATGAAT ATCGAAAGCT TGTGAGGAAC GGAAAACTTG CTTGCACCAG AGAGAACGAT 1200
   CCTATTCAGG GCCCAGATGG GAAAGTGCAC GGCAACACCT GCTCCATGTG TGAGGTTTTT 1260
   TTCCAAGCAG AAGAAGAAGA AAAGAAAAAG AAGGAAGGCG AATCAAGAAA CAAAAGACAA 1320
   TCTAAGAGTA CAGCTTCCTT TGAGGAGTTG TGTAGTGAAT ACCGCAAATC CAGGAAAAAC 1380
   GGACGGCTTT TTTGCACCAG AGAGAATGAC CCCATCCAGG GCCCAGATGG GAAAATGCAT 1440
   GGCAACACCT GCTCCATGTG TGAGGCCTTC TTTCAACAAG AAGAAAGAGC AAGAGCAAAG 1500
   GCTAAAAGAG AAGCTGCAAA GGAAATCTGC AGTGAATTTC GGGACCAAGT GAGGAATGGA 1560
   ACACTTATAT GCACCAGGGA GCATAATCCT GTCCGTGGAC CAGATGGCAA AATGCATGGA 1620
   AACAAGTGTG CCATGTGTGC CAGTGTGTTC AAACTTGAAG AAGAAGAGAA GAAAAATGAT 1680
   AAAGAAGAAA AAGGGAAAGT TGAGGCTGAA AAAGTTAAGA GAGAAGCAGT TCAGGAGCTG 1740
   TGCAGTGAAT ATCGTCATTA TGTGAGGAAT GGACGACTCC CCTGTACCAG AGAGAATGAT 1800
   CCTATTGAGG GTCTAGATGG GAAAATCCAC GGCAACACCT GCTCCATGTG TGAAGCCTTC 1860
   TTCCAGCAAG AAGCAAAAGA AAAAGAAAGA GCTGAACCCA GAGCAAAAGT CAAAAGAGAA 1920
   GCTGAAAAGG AGACATGCGA TGAATTTCGG AGACTTTTGC AAAATGGAAA ACTTTTCTGC 1980
   ACAAGAGAAA ATGATCCTGT GCGTGGCCCA GATGGCAAGA CCCATGGCAA CAAGTGTGCC 2040
   ATGTGTAAGG CAGTCTTCCA GAAAGAAAAT GAGGAAAGAA AGAGGAAAGA AGAGGAAGAT 2100
   CAGAGAAATG CTGCAGGACA TGGTTCCAGT GGTGGTGGAG GAGGAAACAC TCAGGACGAA 2160
   TGTGCTGAGT ATCGGGAACA AATGAAAAAT GGAAGACTCA GCTGTACTCG GGAGAGTGAT 2220
   CCTGTACGTG ATGCTGATGG CAAATCGTAC AACAATCAGT GTACCATGTG TAAAGCAAAA 2280
   TTGGAAAGAG AAGCAGAGAG AAAAAATGAG TATTCTCGCT CCAGATCAAA TGGGACTGGA 2340
   TCAGAATCAG GGAAGGATAC ATGTGATGAG TTTAGAAGCC AAATGAAAAA TGGAAAACTT 2400
   ATCTGCACTC GAGAAAGTGA CCCTGTCCGG GGTCCAGATG GCAAGACACA TGGTAATAAG 2460
   TGTACTATGT GTAAGGAAAA ACTGGAAAGG GAAGCAGCTG AAAAAAAAAG AAAGAGGATG 2520
   AAGACAGGAG CAATACAGGA GAAAGGAGCA ATACAGGAGA AAGGAGCAAT GACAAAGAGG 2580
   ATCTGTGTCG TGAATTTCGA AGCATGCAGA GAAATGGAAA GCTTATCTGC ACCAGAGAAA 2640
   ATAACCCTGT TCGAGGCCCA TATGGCAAGA TGCACATCAA TAAATGTGCT ATGTGTCAGA 2700
   GCATCTTTGA TCGAGAAGCT AATGAAAGAA AAAAGAAAGA TGAAGAGAAA TCAAGTAGCA   2760
   AGCCCTCAAA TAATGCAAAG GATGAGTGCA GTGAATTTCG AAACTATATA AGGAACAATG   2820
   AACTCATCTG CCCTAGAGAG AATGACCCAG TGCACGGTGC TGATGGAAAG TTCTATACAA   2880
   ACAAGTGCTA CATGTGCAGA GCTGTCTTTC TAACAGAAGC TTTGGAAAGG GCAAAGCTTC   2940
   AAGAAAAACC ATCCCATGTT AGAGCTTCTC AAGAGGAAGA CAGCCCAGAC TCTTTCAGTT   3000
   CTCTGGATTC TGAGATGTGC AAAGACTACC GAGTATTGCC CAGGATAGGC TATCTTTGTC   3060
   CAAAGGATTT AAAGCCTGTC TGTGGTGACG ATGGCCAAAC CTACAACAAT CCTTGCATGC   3120
   TCTGTCATGA AAACCTGATA CGCCAAACAA ATACACACAT CCGCAGTACA GGGAAGTGTG   3180
   AGGAGAGCAG CACCCCAGGA ACCACCGCAG CCAGCATGCC CCCGTTTGAC GAATGACAGG   3240
   AAGATTGTTG AAAGCCATGA GGGAAAAAAT AAACCCCAGT TTTGAATCAC CTACCTTCAC   3300
   CATCTGTATA TACAAAGAAT TTTTCGGAGC TTGTTTTATT TGCTATAGAA AACAATACAG   3360
   AGCTTTTGGG AATGGAATCA CTGATTTTCA GTCTTTTCCA TTTCTTTCCT CCTAGAATCT   3420
   GTGATCTGAG GGTATAAAGA CATTTCCACC AAGTTTGAGC CCTCAAAATG TCCTGATTAC   3480
   AATGCTGTCT GTCCAACTGC CTGTTCAATA AAAGTAAACT CAGCAGAAAA A            3531
(2) ANGABEN ZU SEQ ID NO: 7:
   (i) SEQUENZKENNZEICHEN:
      (A) LÄNGE: 13 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 7:
   His Glu Phe Gln Ala Phe Met Lys Asn Gly Lys Leu Phe
   1               5                   10
(2) ANGABEN ZU SEQ ID NO: 8:
   (i) SEQUENZKENNZEICHEN:
      (A) LÄNGE: 13 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 8:
   Ser Glu Tyr Arg Lys Ser Arg Lys Asn Gly Arg Leu Phe
   1 5 10
(2) ANGABEN ZU SEQ ID NO: 9:
   (i) SEQUENZKENNZEICHEN:
      (A) LÄNGE: 13 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 9:
   Asp Asp Phe Lys Lys Gly Glu Arg Asp Gly Asp Phe Ile
   1 5 10
(2) ANGABEN ZU SEQ ID NO: 10:
   (i) SEQUENZKENNZEICHEN:
      (A) LÄNGE: 13 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 10:
   Ser Glu Phe Arg Asp Gln Val Arg Asn Gly Thr Leu Ile
   1 5 10
(2) ANGABEN ZU SEQ ID NO: 11:
   (i) SEQUENZKENNZEICHEN:
      (A) LÄNGE: 13 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 11:
   Ser Ala Phe Arg Pro Phe Val Arg Asn Gly Arg Leu Gly
   1 5 10
(2) ANGABEN ZU SEQ ID NO: 12:
   (i) SEQUENZKENNZEICHEN:
      (A) LÄNGE: 13 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 12:
   Ser Glu Tyr Arg His Tyr Val Arg Asn Gly Arg Leu Pro
   1               5                   10
(2) ANGABEN ZU SEQ ID NO: 13:
   (i) SEQUENZKENNZEICHEN:
      (A) LÄNGE: 13 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 13:
   Lys Glu Tyr Glu Lys Gln Val Arg Asn Gly Arg Leu Phe
   1               5                   10
(2) ANGABEN ZU SEQ ID NO: 14:
   (i) SEQUENZKENNZEICHEN:
      (A) LÄNGE: 13 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 14:
   Asp Glu Phe Arg Arg Leu Leu Gln Asn Gly Lys Leu Phe
   1               5                   10
(2) ANGABEN ZU SEQ ID NO: 15:
   (i) SEQUENZKENNZEICHEN:
      (A) LÄNGE: 13 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 15:
   Ser Gln Tyr Gln Asn Gln Ala Lys Asn Gly Ile Leu Phe
   1               5                   10
(2) ANGABEN ZU SEQ ID NO: 16:
   (i) SEQUENZKENNZEICHEN:
      (A) LÄNGE: 13 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 16:
   Ala Glu Tyr Arg Glu Gln Met Lys Asn Gly Arg Leu Ser
   1               5                   10
(2) ANGABEN ZU SEQ ID NO: 17:
   (i) SEQUENZKENNZEICHEN:
      (A) LÄNGE: 13 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 17:
   Asn Glu Tyr Arg Lys Leu Val Arg Asn Gly Lys Leu Ala
   1               5                   10
(2) ANGABEN ZU SEQ ID NO: 18:
   (i) SEQUENZKENNZEICHEN:
      (A) LÄNGE: 13 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 18:
   Asp Glu Phe Arg Ser Gln Met Lys Asn Gly Lys Leu Ile
   1               5                   10
(2) ANGABEN ZU SEQ ID NO: 19:
   (i) SEQUENZKENNZEICHEN:
      (A) LÄNGE: 18 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 19:
   Pro Gln Asp Lys Lys Phe Phe Gln Ser Leu Asp Gly Ile Met Phe Ile
   1               5                   10                  15
   Asn Lys
(2) ANGABEN ZU SEQ ID NO: 20:
   (i) SEQUENZKENNZEICHEN:
      (A) LÄNGE: 18 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 20:
   Thr Arg Glu Asn Asp Pro Ile Gln Gly Pro Asp Gly Lys Met His Gly
   1               5                   10                  15
   Asn Thr
(2) ANGABEN ZU SEQ ID NO: 21:
   (i) SEQUENZKENNZEICHEN:
      (A) LÄNGE: 18 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 21:
   Thr Arg Glu Asn Asp Pro Val Leu Gly Pro Asp Gly Lys Thr His Gly
   1               5                   10                  15
   Asn Lys
(2) ANGABEN ZU SEQ ID NO: 22:
   (i) SEQUENZKENNZEICHEN:
      (A) LÄNGE: 18 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 22:
   Thr Arg Glu His Asn Pro Val Arg Gly Pro Asp Gly Lys Met His Gly
   1               5                   10                  15
   Asn Lys
(2) ANGABEN ZU SEQ ID NO: 23:
   (i) SEQUENZKENNZEICHEN:
      (A) LÄNGE: 18 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 23:
   Thr Arg Glu Ser Asp Pro Val Arg Gly Pro Asp Gly Arg Met His Gly
   1               5                   10                  15
   Asn Lys
(2) ANGABEN ZU SEQ ID NO: 24:
   (i) SEQUENZKENNZEICHEN:
      (A) LÄNGE: 18 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 24:
   Thr Arg Glu Asn Asp Pro Ile Glu Gly Leu Asp Gly Lys Ile His Gly
   1               5                   10                  15
   Asn Thr
(2) ANGABEN ZU SEQ ID NO: 25:
   (i) SEQUENZKENNZEICHEN:
      (A) LÄNGE: 18 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 25:
   Thr Arg Glu Asn Asp Pro Ile Arg Gly Pro Asp Gly Lys Met His Gly
   1               5                   10                  15
   Asn Leu
(2) ANGABEN ZU SEQ ID NO: 26:
   (i) SEQUENZKENNZEICHEN:
      (A) LÄNGE: 18 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 26:
   Thr Arg Glu Asn Asp Pro Val Arg Gly Pro Asp Gly Lys Thr His Gly
   1               5                   10                  15
   Asn Lys
(2) ANGABEN ZU SEQ ID NO: 27:
   (i) SEQUENZKENNZEICHEN:
      (A) LÄNGE: 18 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 27:
   Thr Arg Glu Asn Asp Pro Ile Gln Gly Pro Asp Gly Lys Val His Gly
   1               5                   10                  15
   Asn Thr
(2) ANGABEN ZU SEQ ID NO: 28:
   (i) SEQUENZKENNZEICHEN:
      (A) LÄNGE: 18 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 28:
   Thr Arg Glu Ser Asp Pro Val Arg Asp Ala Asp Gly Lys Ser Tyr Asn
   1               5                   10                  15
   Asn Gln
(2) ANGABEN ZU SEQ ID NO: 29:
   (i) SEQUENZKENNZEICHEN:
      (A) LÄNGE: 18 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 29:
   Thr Arg Glu Ser Asp Pro Val Arg Gly Pro Asp Gly Lys Thr His Gly
   1               5                   10                  15
   Asn Lys
(2) ANGABEN ZU SEQ ID NO: 30:
   (i) SEQUENZKENNZEICHEN:
      (A) LÄNGE: 2 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 30:
   Ala Thr
   1
(2) ANGABEN ZU SEQ ID NO: 31:
   (i) SEQUENZKENNZEICHEN:
      (A) LÄNGE: 2 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 31:
   Ala Leu
   1
(2) ANGABEN ZU SEQ ID NO: 32:
   (i) SEQUENZKENNZEICHEN:
      (A) LÄNGE: 2 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 32:
   Ala Met
   1
(2) ANGABEN ZU SEQ ID NO: 33:
   (i) SEQUENZKENNZEICHEN:
      (A) LÄNGE: 2 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 33:
   Ser Met
   1
(2) ANGABEN ZU SEQ ID NO: 34:
   (i) SEQUENZKENNZEICHEN:
      (A) LÄNGE: 2 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (xi) SEQUENZBESCHREIBUNG: SEQ ID NO: 34:
   Thr Met
   1